# EUROPEAN PATENT SPECIFICATION

(11) **EP 1 183 072 B1**
(45) Date of publication and mention of the grant of the patent: **11.08.2010**
(21) Application number: 00935404.4
(22) Date of filing: 05.06.2000
(51) Int. Cl.: A61N 5/06

(54) **TISSUE REJUVENATION BY ILLUMINATING RADIATION**
GEWEBEREGENERIERUNG MITTELS LICHTBESTRAHLUNG
RAJEUNISSEMENT DES TISSUS PAR RAYONNEMENT LUMINEUX

(30) Priority: 04.06.1999 GB 9912877
(43) Date of publication of application: 06.03.2002
(73) Proprietor: ICN PHOTONICS LIMITED, LLanelli, Carmarthenshire SA14 8QG (GB)
(72) Inventor: Kiernan, Michael, Noel, Blackpill, Swansea SA3 5BL (GB); Clement, Robert, Marc, Pontardawe SA8 3HD (GB); Bjerring, Peter, DK-8240 Risskov (DK)
(74) Representative: Davies, Gregory Mark
(86) International application number: PCT/GB2000/002171
(87) International publication number: WO 2000/074782

(56) References cited:
- EP-A- 0 763 371
- WO-A-98/24512
- WO-A-98/38933
- DE-A- 4 212 393
- US-A- 5 196 005
- US-A- 5 810 801

## Description

The present invention relates to tissue rejuvenation and in particular to tissue rejuvenation by means of selective production of collagen at a target site.

The human body has a variety of different types of collagen, which essentially constitute the extracellular matrix of the body. This matrix is the material that binds and supports cells and is essential for the survival of a multicellular organism. Collagens provide the tissue with tensile strength.

The various collagen containing structures in the body include bone, dentin, cartilage, uterus and the larger vessels in the circulatory system. As the body ages the rate of collagen naturally decreases leading to breakdown in tissue and organ structure and function. Other problems can also exacerbate or cause tissue or organ structure deterioration due to inhibition of collagen formation.

According to the invention there is provided apparatus for stimulating collagen containing structures, as set out in Claim 1. The apparatus involves illuminating a target structure with illuminating radiation causing elevation of the temperature of a target structure, the radiation dosed to the target being controlled to induce a predetermined and precise inflammatory response in the target tissue. The absorption of the radiation by the target structure at the predetermined low level controlled dose (resulting in the inflammatory response of the target structure tissue) stimulates collagen regrowth.

It is important that the radiation dose is controlled to ensure that overheating of the target tissue structure does not take place. Overdosing of energy (radiation) leads to thermal damage inhibiting the inflammatory phase resulting in less than optimum collagen formation. It is important therefore that the radiation energy dose delivered is of sufficiently low intensity and power to avoid tissue destruction. The radiation dose is therefore controlled dependent upon the body structure or tissue being illuminated but in all cases the intensity and duration of the illuminating radiation is relatively low level to prevent damage of the target structure or tissue.

The wavelength of the illuminating radiation is selected such that there is at least some absorption by the target structure or tissue.

The illuminating radiation may be generated by laser, laser diode, light emitting diode, or a broad band white light source. The illuminating radiation is of a discrete wavelength . For a broad band white light source an appropriate filter is preferably provided.

Where the illuminating radiation is laser radiation, the laser may, for example comprises pulsed dye laser (585nm).

LED's at wavelengths substantially in the range 550-1000nm would be suitable.

The technique can be used on a variety of body tissue structures either by means of direct external illumination of structures or by means of directing the illuminating radiation into the body (for example along a suitable waveguide) to be delivered to the site of the internal target structure.

According to a second aspect, there is provided apparatus for use in effecting refurbishment of tissue and/or tissue structures, which apparatus includes:
i) a source of illuminating radiation; and,
ii) means for directing the illuminating radiation to a target site.

The means for directing the illuminating radiation to the target site preferably includes focussing means (for example optical focussing means). The means for directing the illuminating radiation to the target site preferably includes a flexible optical line including a distal portion through which the radiation is emitted in order to illuminate the target structure. The optical line may comprise an optical waveguide such as a length of fibreoptic.

The means for directing the illuminating radiation to the target site is preferably configured to permit manual manipulation enabling the zone of radiation impingement with the target site to be manually altered. Alternatively, the apparatus may be provided with an automated drive arrangement.

Desirably, the illuminating radiation is pulsed, having a pulse duration of 350 µs . Alternatively, scanning of illuminating radiation can produce a similar effect, in that localised tissue is irradiated only for the required short time period to deliver the appropriate energy dose.

The invention will now be further described in specific embodiments by way of example only, and with reference to the accompanying drawings, in which:
Figure 1 is a schematic representation of a first embodiment of a technique according to the invention,
Figure 2 is a schematic representation of a second embodiment of a technique according to the invention;
Figure 3 is a schematic representation of a further embodiment of a technique according to the invention;
Figure 4 is a schematic representation of a further embodiment of a technique according to the invention;
Figure 5 is a schematic representation of a further embodiment of a technique according to the invention; and,
Figure 6 is a schematic representation of a further embodiment of a technique according to the invention.

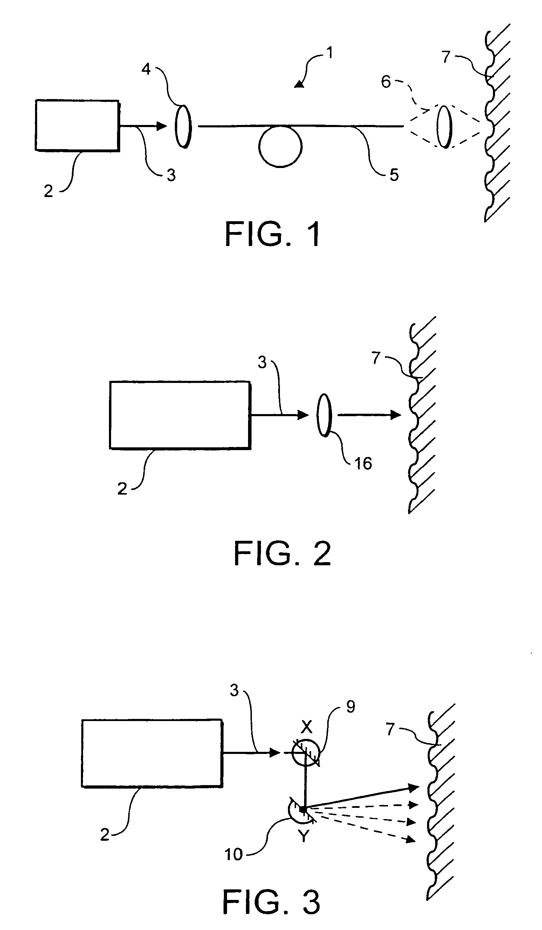

Referring to the drawings, and initially to Figure 1, there is shown a tissue rejuvenation technique in which an arrangement 1 includes a light source 2, such as an LED, laser diode or other laser or a white light source (provided with an appropriate filter), having a wavelength in a narrow bandwidth in the range 550-1000nm, directs a beam 3 via focussing optics 4 into a fibreoptic waveguide 5. Light emitted from the distal end of fibreoptic waveguide 5 passes through a collimating lens 6 where it is directed to illuminate the surface of a tissue structure 7.

In the embodiment shown in Figure 2, the beam 3 emitted from light source 2 passes directly through a focussing lens 16 which focusses the beam onto the tissue structure 7.

In the embodiment shown in Figure 3, the beam 3 from the light source 2 is directed to a scanning optical arrangement comprising rotating scanning mirrors 9, 10 arranged to scan the beam in orthogonal X-Y directions onto the tissue 7.

In each of the embodiments shown in Figures 1 to 3, the relevant tissue structure 7 is directly illuminated from externally of the body (extra-corporeal illumination).

The intensity and duration of the light beam illuminating the tissue 7 is controlled such that the energy dosed to the tissue is at a level where collagen formation is promoted, without the tissue being "injured" to a degree at which structural integrity of the tissue deteriorates. Illumination promoting collagen production mirroring wound healing in the inflammatory, proliferate and remodelling phases results in collagen production and enhancement of the structural integrity of the tissue.

It is important that the wavelength of the light illuminating the tissue is selected to have at least a component which is selectively absorbed to the required degree by the tissue in question. Appropriate selection of the wavelength to be absorbed by the tissue, or a chromophore at or below the tissue surface, can enable discrete target sites at or below the tissue surface to be targeted.

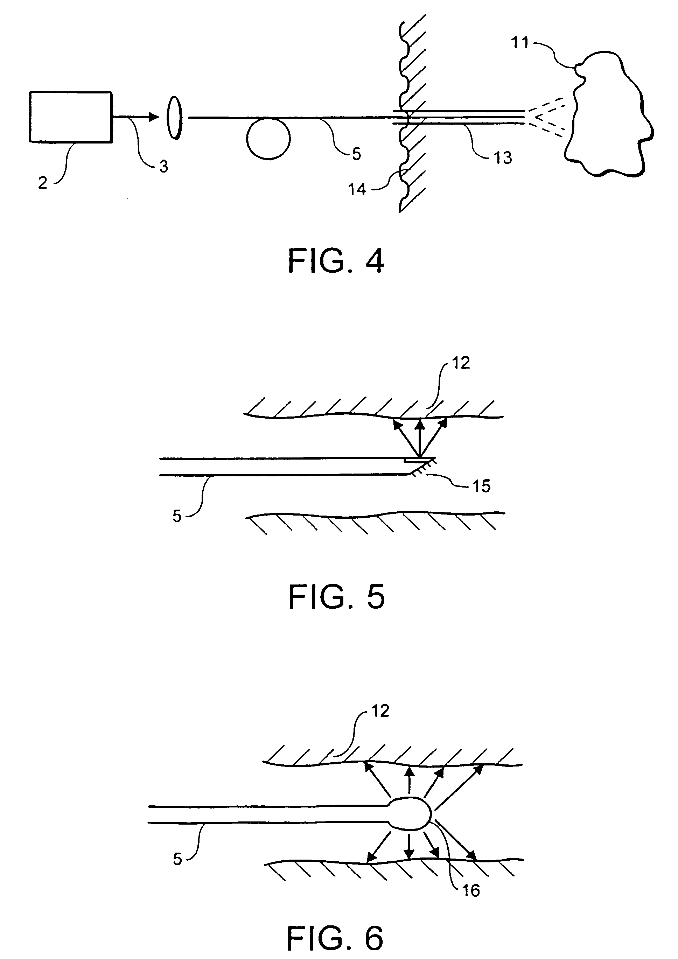

The arrangements shown in Figure 4 to 6 relate to interstitial rejuvenation techniques where an extra corporeal light source 2 produces a beam 3 which can be directed through the body surface interface to target an internal cell structure 11, 12.

In the embodiment shown in Figure 4, the light beam 3 is focussed into a fibreoptic waveguide 5 which extends through a sheathing catheter 13. Light emanating from the end of fibreoptic waveguide 5 illuminates the target structure 11 below the body surface 14.

In the embodiments shown in Figures 5 and 6, the fibreoptic waveguide 5 extends into and along a target vessel 12 which comprises the circulatory system of the body (such as for example an artery).

In the embodiment of Figure 5, light is reflected from a mirror end 15 to illuminate the desired "target" portion of the internal vessel wall 12.

In the embodiment shown in Figure 6, the fibreoptic waveguide 5 is provided with a diffusing end 16 arranged to diffuse the light to illuminate radially the entire "target" portion of the vessel wall 12.

Illumination of the relevant tissue target structure with illuminating radiation of the required wavelength and dosage produces the inflammatory/wound healing response promoting the maximum degree of collagen formation. Promotion of collagen at the target site effectively rejuvenates the target tissue/structure.

To test the efficacy of the invention, examples have been performed using laser radiation and measuring terminals of Type III collagen produced.

When Type III collagen is formed, the molecule is in the form of a long chain with two terminals on either end. When three collagen molecules have been produced, the molecules bond together and the terminals on either end of the chain separate and are released into the dermal interstitial fluid. By measuring the quantity of the terminals in the fluid, the rate of collagen production can be measured.

As an example of the technique, biochemical investigations have shown that the production rate of Type III collagen in the dermal region of the skin can be increased by the application of the following laser parameters according to the invention:

| | |
|---|---|
| Wavelength: | 585nm |
| Pulse Duration: | 350µsec |
| Energy Density: | 2.4J/cm² |
| Spot Size: | 5mm Diameter |

delivered to the surface of the skin via a flexible fibre optic.

When the skin was irradiated with these parameters, the production rate of Type III collagen in the skin increased by 84%.

Similar tests performed with a Frequency Doubled Nd:YAG laser operating at the following parameters not forming part of the present invention:

| | |
|---|---|
| Wavelength: | 532nm |
| Pulse Duration: | 2msec to 20msec |
| Energy Density: | 2-20J/cm² |
| Spot Size: | 3mm (dia) |

showed an increase of between 22% and 44% in the Type III Collagen production rate.

Both tests were performed by treating a selected area of skin with the prescribed laser parameters and waiting 72 hours before raising suction blisters on the treated and untreated control areas. The interstitial fluid collected from the suction blisters was analysed using an immunoflourescent technique to measure the quantity of the amino-propeptide terminal of the Type III collagen molecule (PIIINP).

The quantity of PIIINP found in the blister fluid is related to the amount of Type III collagen being produced at the investigation site. The percentage increase figures quoted are relative to adjacent control sites.

## Claims

1. Apparatus for stimulating collagen containing structures, said apparatus including a source (2) of illuminating radiation for illuminating a target structure (7) with illuminating radiation and means (6) for directing the illuminating radiation to the target structure, said means including an emitter portion to deliver radiation to the target and a controller to deliver said illuminating radiation to the target structure at a controlled level to induce an inflammatory response in the target tissue to stimulate collagen regrowth without overdosing said target tissue, **characterised in that** the energy density of the illuminating radiation delivered to the target structure (7) is 2.4J/cm², the wavelength of the radiation is 585nm, the spot size of the radiation is 5mm diameter and the pulse duration of the radiation is 350µsec.

2. Apparatus according to claim 1, wherein the means for directing the illuminating radiation to the target site includes:
(a) focussing means; and/or,
(b) an optical delivery line; and,
(c) the emitter portion comprising the optical delivery line or associated therewith through which the radiation is emitted in order to illuminate the target structure.

3. Apparatus according to claim 1 or claim 2, wherein the means for directing the illuminating radiation to the target site is configured to permit manual manipulation enabling the zone of radiation impingement with the target site to be manually altered.

4. Apparatus according to any of claims 1 to 3, wherein the apparatus is provided with an automated drive arrangement.

5. Apparatus according to any of claims 1 to 3, including scanning means (9, 10) for scanning the illuminating radiation over the target tissue structure.

6. Apparatus according to any preceding claim, wherein the illuminating radiation is of a relatively broad band light source filtered to a discrete or relatively narrow wavelength bandwidth.

7. Apparatus according to any preceding claim, wherein the illuminating radiation is laser radiation.

8. Apparatus according to any preceding claim, wherein the illuminating radiation is obtained from an LED.

9. Apparatus according to any preceding claim, wherein the illuminating radiation is obtained from a broad band white light source.

10. Apparatus according to any preceding claim, adepted to illuminate a body tissue structure by means of direct
external illumination of the structure.

11. Apparatus according to any preceding claim, wherein the illuminating radiation is delivered via a fibre optic waveguide (5) to enable the illuminating radiation to illuminate a target structure (11), below a body surface (14).

12. Apparatus according to any preceding claim adapted to induce a controlled inflammatory response in one or more of the following collagen containing structures:
bone
dentine
cartilage
uterus
large veins and arteries.

## Patentansprüche

1. Vorrichtung zur Stimulation von kollagenhaltigen Strukturen, wobei die Vorrichtung eine Quelle (2) für Beleuchtungsstrahlung zum Bestrahlen einer Zielstruktur (7) mit Beleuchtungsstrahlung und Mittel (6) zum Richten der Beleuchtungsstrahlung auf die Zielstruktur enthält, wobei das Mittel einen Strahlerteil zum Abgeben von Strahlung an das Ziel und eine Steuerung zum Abgeben der Beleuchtungsstrahlung an die Zielstruktur mit einem gesteuerten Pegel zum Induzieren einer Entzündungsreaktion in dem Zielgewebe zur Stimulation von Kollagennachwuchs, ohne das Zielgewebe überzudosieren, enthält, **dadurch gekennzeichnet, dass** die Energiedichte der Beleuchtungsstrahlung, die an die Zielstruktur (7) abgegeben wurde, 2,4 J/cm², die Wellenlänge der Strahlung 585 nm, die Fleckengröße der Strahlung 5 mm Durchmesser und die Impulsdauer der Strahlung 350 µs beträgt.

2. Vorrichtung nach Anspruch 1, wobei das Mittel zum Richten der Beleuchtungsstrahlung auf den Zielort Folgendes enthält:
(a) Fokussiermittel; und/oder
(b) eine optische Abgabeleitung; und
(c) den Strahlerteil, der die optische Abgabeleitung umfasst oder damit verbunden ist und durch den die Strahlung abgegeben wird, um die Zielstruktur zu bestrahlen.

3. Vorrichtung nach Anspruch 1 oder Anspruch 2, wobei das Mittel zum Richten der Beleuchtungsstrahlung auf den Zielort dazu konfiguriert ist, manuelle Manipulationen zu gestatten, die es ermöglichen, die Zone des Strahlungseinfalls mit dem Zielort manuell zu ändern.

4. Vorrichtung nach einem der Ansprüche 1 bis 3, wobei die Vorrichtung mit einer automatisierten Antriebseinrichtung versehen ist.

5. Vorrichtung nach einem der Ansprüche 1 bis 3, enthaltend Scan-Mittel (9, 10) zum Führen der Beleuchtungsstrahlung über die Zielgewebestruktur.

6. Vorrichtung nach einem vorhergehenden Anspruch, wobei die Beleuchtungsstrahlung von einer relativ breitbandigen Lichtquelle stammt und auf eine diskrete oder relativ schmale Wellenlängenbandbreite gefiltert ist.

7. Vorrichtung nach einem vorhergehenden Anspruch, wobei es sich bei der Beleuchtungsstrahlung um Laserstrahlung handelt.

8. Vorrichtung nach einem vorhergehenden Anspruch, wobei die Beleuchtungsstrahlung von einer LED erhalten wird.

9. Vorrichtung nach einem vorhergehenden Anspruch, wobei die Beleuchtungsstrahlung von einer breitbandigen Weißlichtquelle erhalten wird.

10. Vorrichtung nach einem vorhergehenden Anspruch, ausgeführt zum Bestrahlen einer Körpergewebestruktur mithilfe von direkter externer Bestrahlung der Struktur.

11. Vorrichtung nach einem vorhergehenden Anspruch, wobei die Beleuchtungsstrahlung über einen faseroptischen Wellenleiter (5) abgegeben wird, damit die Beleuchtungsstrahlung eine Zielstruktur (11) unter einer Körperoberfläche (14) bestrahlen kann.

12. Vorrichtung nach einem vorhergehenden Anspruch, ausgeführt zum Induzieren einer gesteuerten Entzündungsreaktion in einer oder mehreren der folgenden kollagenenthaltenden Strukturen:
Knochen
Zahnbein
Knorpel
Gebärmutter
große Venen und Arterien.

## Revendications

1. Appareil destiné à stimuler des structures contenant du collagène, ledit appareil comprenant une source (2) de rayonnement d'illumination destinée à illuminer une structure cible (7) avec un rayonnement d'illumination et un moyen (6) destiné à diriger le rayonnement d'illumination vers la structure cible, ledit moyen comprenant une partie émettrice destinée à délivrer un rayonnement à la cible et une unité de commande destinée à délivrer ledit rayonnement d'illumination vers la structure cible à un niveau commandé afin d'induire une réponse inflammatoire dans le tissu cible pour stimuler la régénération du collagène sans surdosage dudit tissu cible, **caractérisé en ce que** la densité d'énergie du rayonnement d'illumination délivré à la structure cible (7) est de 2,4 J/cm², la longueur d'onde du rayonnement est de 585 nm, la taille du point lumineux du rayonnement correspond à un diamètre de 5 mm et la durée d'impulsion du rayonnement est de 350 µs.

2. Appareil selon la revendication 1, dans lequel le moyen destiné à diriger le rayonnement d'illumination vers le site cible comprend :
(a) un moyen de focalisation ; et/ou
(b) une ligne optique d'apport ; et
(c) la partie émettrice comprenant la ligne optique d'apport ou associée à celle-ci, par l'intermédiaire de laquelle le rayonnement est émis afin d'illuminer la structure cible.

3. Appareil selon la revendication 1 ou la revendication 2, dans lequel le moyen destiné à diriger le rayonnement d'illumination vers le site cible est configuré pour permettre une manipulation manuelle autorisant une altération manuelle de la zone d'incidence du rayonnement sur le site cible.

4. Appareil selon l'une quelconque des revendications 1 à 3, dans lequel l'appareil est muni d'un système d'attaque automatisée.

5. Appareil selon l'une quelconque des revendications 1 à 3, comprenant un moyen de balayage (9, 10) destiné à animer d'un mouvement de balayage le rayonnement d'illumination sur la structure du tissu cible.

6. Appareil selon l'une quelconque des revendications précédentes, dans lequel le rayonnement d'illumination est une source de lumière à bande relativement large filtrée à une largeur de bande de longueur d'onde discrète ou relativement étroite.

7. Appareil selon l'une quelconque des revendications précédentes, dans lequel le rayonnement d'illumination est un rayonnement laser.

8. Appareil selon l'une quelconque des revendications précédentes, dans lequel le rayonnement d'illumination est obtenu à partir d'une LED.

9. Appareil selon l'une quelconque des revendications précédentes, dans lequel le rayonnement d'illumination est obtenu à partir d'une source de lumière blanche à large bande.

10. Appareil selon l'une quelconque des revendications précédentes, conçu pour illuminer la structure d'un tissu corporel par illumination externe directe de la structure.

11. Appareil selon l'une quelconque des revendications précédentes, dans lequel le rayonnement d'illumination est délivré par l'intermédiaire d'un guide d'ondes à fibre optique (5) pour permettre au rayonnement d'illumination d'illuminer une structure cible (11) en dessous d'une surface corporelle (14).

12. Appareil selon l'une quelconque des revendications précédentes, conçu pour induire une réponse inflammatoire régulée dans une ou plusieurs des structures suivantes contenant du collagène :
os
dentine
cartilage
utérus
grandes veines et artères.
